# EUROPEAN PATENT APPLICATION

(11) **EP 4 671 288 A1**
(43) Date of publication of application: **31.12.2025**
(21) Application number: 23924261.3
(22) Date of filing: 26.12.2023
(51) Int. Cl.: C08F 20/30, C07C 69/54, C07C 271/48

(54) **FLUORENE SKELETON-CONTAINING (METH)ACRYLATE COMPOUND, RESIN COMPOSITION, AND CURED PRODUCT**

(30) Priority: 24.02.2023 JP 2023027507
(71) Applicant: SHIN-ETSU CHEMICAL CO., LTD., Tokyo 100-0005 (JP)
(72) Inventor: OMORI, Hiroto, Annaka-shi, Gunma 379-0224 (JP); KAWAGUCHI, Yugo, Annaka-shi, Gunma 379-0224 (JP)
(74) Representative: Ter Meer Steinmeister & Partner
(86) International application number: PCT/JP2023/046716
(87) International publication number: WO 2024/176617

(57) **Abstract**

Provided is a fluorene skeleton-containing (meth)acrylate compound containing an alkenyl group having a double bond at the terminal thereof.

## Description

### TECHNICAL FIELD

The present invention relates to a fluorene skeleton-containing (meth)acrylate compound, a resin composition, and a cured product.

### BACKGROUND ART

Polyfunctional (meth)acrylates synthesized from bisphenols are widely used in coating agents, lenses, displays, and the like. In recent years, with the increase in functionality of optical materials, the polyfunctional (meth)acrylates have been required to have improved properties such as heat resistance and refractive index.

Among bisphenols, a 9,9-bisphenol fluorene compound is known to have excellent heat resistance and a high refractive index, and a polyfunctional (meth)acrylate compound derived from such a compound is also used.

For example, Patent Document 1 discloses a lens material that is a composition containing, as a main component, a polyfunctional (meth)acrylate derived from 9,9-bisphenol fluorene represented by the formula (X).

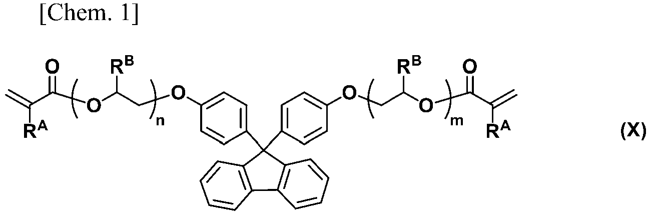

In the formula, R^{A} and R^{B} each represent hydrogen or a methyl group, and m and n each represent an integer of 0 to 5.

In addition, Patent Document 2 discloses a polymerizable composition including a specific polyfunctional (meth)acrylate having a fluorene skeleton, a hydrolytically condensable organosilicon compound having a polymerizable group, a photoacid generator, and a photoradical initiator, and describes, as the polyfunctional (meth)acrylate, a (meth)acrylate of 9,9-bis (mono- to trihydroxyphenyl)fluorene, a (meth)acrylate of an alkylene oxide(C₂₋₄ alkylene oxide) adduct of 9,9-bis (mono- to trihydroxyphenyl)fluorene, and the like.

While the use of these compounds can improve the heat resistance and refractive index of the resin, there is a problem that the introduction of a rigid fluorene skeleton generally causes a low elongation percentage and gives a brittle resin.

### PRIOR ART DOCUMENTS

### PATENT DOCUMENTS

Patent Document 1: JP 3130555
Patent Document 2: JP 4756977

### SUMMARY OF INVENTION

### TECHNICAL PROBLEM

The present invention has been made in view of the above circumstances, and an object thereof is to provide a novel fluorene skeleton-containing (meth)acrylate compound having a higher elongation percentage of a cured product than that of a conventional fluorene skeleton-containing (meth)acrylate compound, a resin composition containing the compound, and a cured product obtained from the resin composition.

### SOLUTION TO PROBLEM

As a result of extensive studies to achieve the above object, the present inventors have found that a fluorene skeleton-containing (meth)acrylate compound in which an alkenyl group having a double bond is introduced at a terminal thereof improves the elongation of a cured product as compared with the case of using a conventional fluorene skeleton-containing (meth)acrylate compound, and have completed the present invention.

Accordingly, the present invention provides the following fluorene skeleton-containing (meth)acrylate compound, resin composition, and cured product.
1. A fluorene skeleton-containing (meth)acrylate compound containing an alkenyl group having a double bond at a terminal thereof.
2. The fluorene skeleton-containing (meth)acrylate compound according to 1, which has the following formula (1): 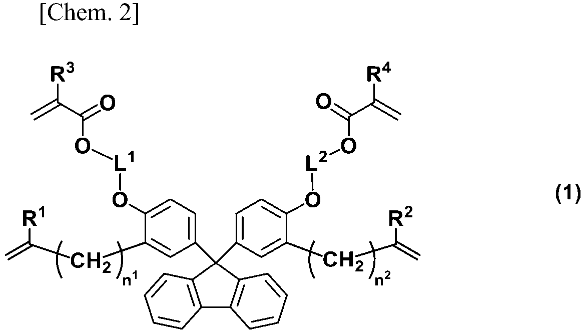 wherein n¹ and n² are each independently an integer of 1 to 7;
   R¹ to R⁴ are each independently a hydrogen atom or a methyl group; and
   L¹ and Lare each independently a saturated hydrocarbylene group having 1 to 15 carbon atoms, where a part of -CH₂- of the saturated hydrocarbylene group may be substituted with -O-, -S-, -SO₂-, -CO-, or -CONH-, and a part or all of hydrogen atoms of the saturated hydrocarbylene group may be substituted with a hydroxy group.
3. The fluorene skeleton-containing (meth)acrylate compound according to 2, wherein L¹ and L² are both saturated hydrocarbylene groups in which one or more hydrogen atoms are substituted with a hydroxy group or saturated hydrocarbylene groups in which one or more -CH₂- are substituted with -CONH-.
4. The fluorene skeleton-containing (meth)acrylate compound according to 2 or 3, wherein n¹ and n² are both 1.
5. The fluorene skeleton-containing (meth)acrylate compound according to any one of 2 to 4, wherein both L¹ and L² have 1 to 8 carbon atoms.
6. A resin composition containing the fluorene skeleton-containing (meth)acrylate compound according to any one of 1 to 5 and a polymerization initiator.
7. A cured product obtained from the resin composition according to 6.

### ADVANTAGEOUS EFFECTS OF INVENTION

The fluorene skeleton-containing (meth)acrylate compound of the present invention gives a film having an improved elongation percentage as compared with the case of using a fluorene skeleton-containing (meth)acrylate compound that has been conventionally used.

### DESCRIPTION OF EMBODIMENTS

### [Fluorene skeleton-containing (meth)acrylate compound]

The fluorene skeleton-containing (meth)acrylate compound of the present invention contains an alkenyl group having a double bond at the terminal thereof.

Such a compound preferably has the following formula (1).

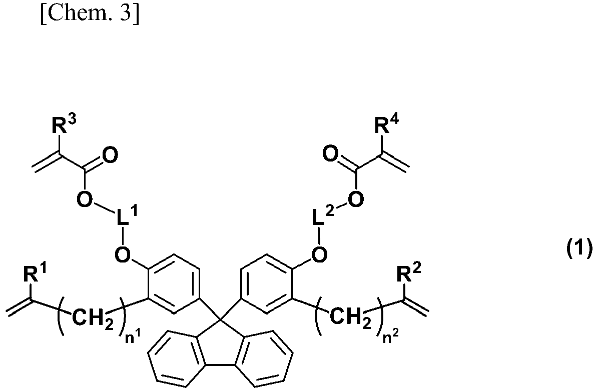

In the formula (1), n¹ and n² are each independently an integer of 1 to 7, more preferably an integer of 1 to 3, and still more preferably 1.

In the formula (1), R¹ to R⁴ are each independently a hydrogen atom or a methyl group, but R¹ and R² are preferably hydrogen atoms.

In the formula (1), L¹ and L² are each independently a saturated hydrocarbylene group having 1 to 15 carbon atoms, where a part of -CH₂- of the saturated hydrocarbylene group may be substituted with -O-, -S-, -SO₂-, -CO-, or -CONH-, and a part or all of hydrogen atoms of the saturated hydrocarbylene group may be substituted with a hydroxy group. Note that -CH₂- of the saturated hydrocarbylene group may be located at the terminal thereof.

The saturated hydrocarbylene group may be linear, branched, or cyclic, and specific examples thereof include alkanediyl groups having 1 to 15 carbon atoms, such as a methanediyl group, an ethane-1,1-diyl group, an ethane-1,2-diyl group, a propane-1,3-diyl group, a butane-1,4-diyl group, a pentane-1,5-diyl group, a hexane-1,6-diyl group, a heptane-1,7-diyl group, an octane-1,8-diyl group, a nonane-1,9-diyl group, a decane-1,10-diyl group, an undecane-1,11-diyl group, a dodecane-1,12-diyl group, a tridecane-1,13-diyl group, a tetradecane-1,14-diyl group, and a pentadecane-1,15-diyl group; and cyclic saturated hydrocarbylene groups having 3 to 15 carbon atoms, such as a cyclopentanediyl group, a cyclohexanediyl group, a norbornanediyl group, and an adamantanediyl group.

Both L¹ and L² preferably have 1 to 8 carbon atoms, and more preferably have one or more hydrogen atoms substituted with a hydroxy group or one or more -CH₂- substituted with -CONH-.

### [Method for producing fluorene skeleton-containing (meth)acrylate compound]

Although a method for producing the fluorene skeleton-containing (meth)acrylate compound of the present invention is not particularly limited, the fluorene skeleton-containing (meth)acrylate compound can be synthesized by a reaction between a 9,9-bisphenol fluorene compound having the formula (2), in which an alkenyl group having a double bond is introduced at the terminal thereof, and a compound having the formula (3).

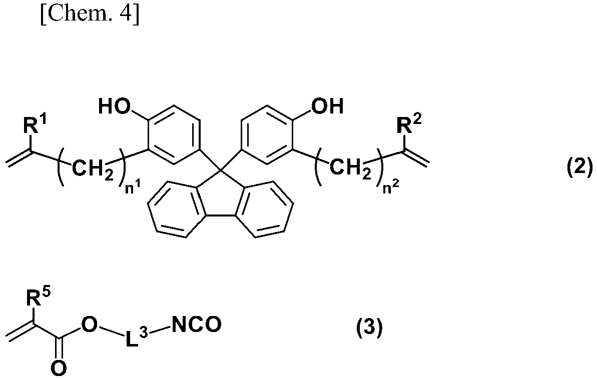

In the formula, n¹, n², R³, and R⁴ are as defined above.

In the formula (3), R⁵ is a hydrogen atom or a methyl group.

In the formula (3), L³ is a saturated hydrocarbylene group having 1 to 14 carbon atoms, where a part of -CH₂- of the saturated hydrocarbylene group may be substituted with -O-, -S-, -SO₂-, -CO-, or -CONH-, and a part or all of hydrogen atoms of the saturated hydrocarbylene group may be substituted with a hydroxy group. The saturated hydrocarbylene group represented by L³ may be linear, branched, or cyclic, but the number of carbon atoms is preferably 1 to 7.

Specific examples of the compound having the formula (3) include 2-acryloyloxyethyl isocyanate (Karenz AOI (registered trademark) manufactured by Showa Denko K.K.), 2-methacryloyloxyethyl isocyanate (Karenz MOI (registered trademark) manufactured by Showa Denko K.K.), and 2-(2-methacryloyloxyethyloxy)ethyl isocyanate (Karenz MOI-EG (registered trademark) manufactured by Showa Denko K.K.), but are not limited thereto.

The reaction conditions are not particularly limited, but usually, the compound having the formula (2) and the compound having the formula (3) may be mixed in a solvent and heated. The solvent to be used is preferably an aprotic polar solvent from the viewpoint of promoting the reaction while suppressing side reactions, and particularly preferred examples thereof include ketones such as cyclopentanone and cyclohexanone; cyclic ethers such as tetrahydrofuran and 1,4-dioxane; and esters such as ethyl acetate and propylene glycol monomethyl ether acetate. The reaction temperature is preferably, for example, 35 to 130°C, particularly preferably 45 to100°C, from the viewpoint of preventing side reactions and enabling completion of the reaction in a short time. The reaction time depends on the type and amount of the reactive substrate, but is preferably about 0.5 to 50 hours, particularly preferably 0.5 to 24 hours.

In the reaction, starting compounds are blended such that a molar ratio of the compound having the formula (3) to the compound having the formula (2) is preferably 1.80 to 2.20, and more preferably 1.95 to 2.10. The compound having the formula (2) and the compound having the formula (3) may each be used singly or in combination of two or more thereof.

In the reaction, a catalyst may be optionally used. Examples of the catalyst include amines such as triethylamine, triethylenediamine, bis-(2-dimethylaminoethyl) ether, and N-methylmorpholine; phosphines such as triphenylphosphine and tri(o-tolyl)phosphine; quaternary ammonium salts such as tetrabutylammonium chloride, benzyltriethylammonium chloride, and tetraethylhydroxylammonium; imidazoles such as imidazole and-2-ethyl-4-methylimidazole; pyridines such as pyridine, N,N-dimethyl-4-aminopyridine, and 2,6-lutidine; organotin compounds such as tin acetate, tin octylate, tin oleate, tin laurate, dibutyltin diacetate, dimethyltin dilaurate, dibutyltin dilaurate, dibutyltin dimercaptide, dibutyltin maleate, dibutyltin dilaurate (dibutyltin (IV) dilaurate), dibutyltin dineodecanoate, dioctyltin dimercaptide, dioctyltin dilaurate, and dibutyltin dichloride; organolead compounds such as lead octanoate and lead naphthenate; organonickel compounds such as nickel naphthenate; organocobalt compounds such as cobalt naphthenate; organocopper compounds such as copper octenoate; organiobismuth compounds such as bismuth octylate and bismuth neodecanoate; and potassium salts such as potassium carbonate, potassium acetate, and potassium octylate.

The catalyst is usually used in a catalytic amount, which is preferably 0.1 to 20 mol% with respect to the compound having the formula (2). The catalyst may be used singly or in combination with two or more thereof.

In the reaction, a polymerization inhibitor may be optionally used. Examples of the polymerization inhibitor that can be used include various phenols, hydroquinones, benzoquinones, catechols, hydroxylamines, nitroso compounds. The amount of the polymerization inhibitor used is not particularly limited, but is preferably 0.001 to 10 wt%, more preferably 0.01 to 5 wt% with respect to the compound having the formula (3).

After completion of the reaction, a solvent is optionally added, and the organic layer is washed with water and then heated under reduced pressure to distill off the solvent, whereupon the compound having the formula (1) is obtained. In addition, at the time of water washing, an aqueous solution of a metal hydroxide such as sodium hydroxide or potassium hydroxide, a metal carbonate such as sodium carbonate, sodium hydrogen carbonate, or potassium carbonate, or a metal hydrogen carbonate may be optionally used.

Another method for producing the compound having the formula (1) also includes a reaction between the compound having the formula (2) and the compound having the formula (4).

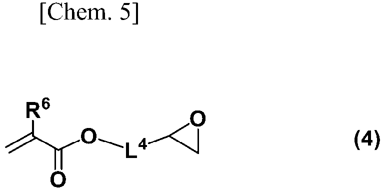

In the formula (4), R⁶ is a hydrogen atom or a methyl group.

In the formula (4), L⁴ is a divalent saturated hydrocarbylene group having 1 to 13 carbon atoms, where a part of -CH₂- of the saturated hydrocarbylene group may be substituted with -O-, -S-, -SO₂-, -CO-, or -CONH-, and a part or all of hydrogen atoms of the saturated hydrocarbylene group may be substituted with a hydroxy group. The saturated hydrocarbylene group represented by L³ may be linear, branched, or cyclic, but the number of carbon atoms is preferably 1 to 6.

The reaction conditions are not particularly limited, but usually, the compound having the formula (2) and the compound having the formula (4) may be mixed in a solvent and heated. The solvent to be used is preferably an aprotic polar solvent from the viewpoint of promoting the reaction while suppressing side reactions, and particularly preferred examples thereof include ketones such as cyclopentanone and cyclohexanone; cyclic ethers such as tetrahydrofuran and 1,4-dioxane; and esters such as ethyl acetate and propylene glycol monomethyl ether acetate. The reaction temperature is preferably, for example, 35 to 130°C, particularly preferably 45 to100°C, from the viewpoint of preventing side reactions and enabling completion of the reaction in a short time. The reaction time depends on the type and amount of the reactive substrate, but is preferably about 0.5 to 50 hours, particularly preferably 0.5 to 24 hours.

In the reaction, starting compounds are blended such that a molar ratio of the compound having the formula (4) to the compound having the formula (2) is preferably 1.60 to 3.00, and more preferably 1.90 to 2.00. The compound having the formula (2) and the compound having the formula (4) may each be used singly or in combination of two or more thereof.

In the reaction, a catalyst may be optionally used. Examples of the catalyst include amines such as triethylamine, triethylenediamine, bis-(2-dimethylaminoethyl) ether, and N-methylmorpholine; phosphines such as triphenylphosphine and tri(o-tolyl)phosphine; quaternary ammonium salts such as tetrabutylammonium chloride, benzyltriethylammonium chloride, and tetraethylhydroxylammonium; imidazoles such as imidazole and 2-ethyl-4-methylimidazole; potassium salts such as potassium hydroxide, potassium carbonate, potassium acetate, and potassium octylate; and sodium salts such as sodium hydroxide, sodium carbonate, sodium acetate, and sodium octylate.

The catalyst is usually used in a catalytic amount, which is preferably 0.1 to 20 mol% with respect to the compound having the formula (2). The catalyst may be used singly or in combination with two or more thereof.

In the reaction, a polymerization inhibitor may be optionally used. Examples of the polymerization inhibitor that can be used include various phenols, hydroquinones, benzoquinones, catechols, hydroxylamines, nitroso compounds. The amount of the polymerization inhibitor used is not particularly limited, but is preferably 0.001 to 10 wt%, more preferably 0.01 to 5 wt% with respect to the compound having the formula (4).

After completion of the reaction, a solvent is optionally added, and the organic layer is washed with water and then heated under reduced pressure to distill off the solvent, whereupon the compound having the formula (1) is obtained. In addition, at the time of water washing, an aqueous solution of a metal hydroxide such as sodium hydroxide or potassium hydroxide, a metal carbonate such as sodium carbonate, sodium hydrogen carbonate, or potassium carbonate, or a metal hydrogen carbonate may be optionally used.

Further, another method for producing the compound having the formula (1) includes a reaction between the compound having the formula (5) and acrylic acid or methacrylic acid.

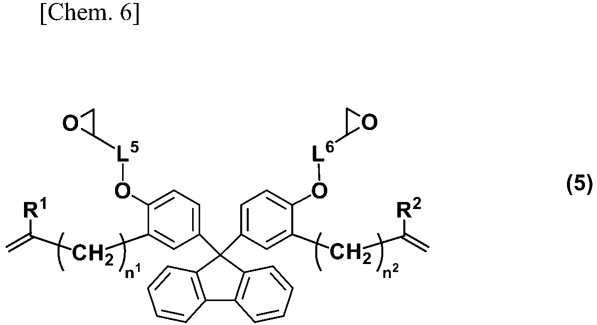

In the formula, n¹, n², R¹, and R² are as defined above.

In the formula (5), L⁵ and L⁶ are each independently a saturated hydrocarbylene group having 1 to 13 carbon atoms, where a part of -CH₂- of the saturated hydrocarbylene group may be substituted with -O-, -S-, -SO₂-, -CO-, or -CONH-, and a part or all of hydrogen atoms of the saturated hydrocarbylene group may be substituted with a hydroxy group. The saturated hydrocarbylene group represented by L⁵ and L⁶ may be linear, branched, or cyclic, but the number of carbon atoms is preferably 1 to 6.

The reaction conditions are not particularly limited, but usually, the compound having the formula (5) and acrylic acid or methacrylic acid may be mixed in a solvent and heated. The solvent to be used is preferably, from the viewpoint of reaction promotion, a polar solvent, and particularly preferably an alcohol-based solvent such as propylene glycol monomethyl ether. The reaction temperature is preferably, for example, 35 to 130°C, particularly preferably 60 to110°C, from the viewpoint of preventing side reactions and enabling completion of the reaction in a short time. The reaction time depends on the type and amount of the reactive substrate, but is preferably about 0.5 to 50 hours, particularly preferably 0.5 to 24 hours.

In the reaction, starting compounds are blended such that a molar ratio of acrylic acid or methacrylic acid to the compound having the formula (5) is preferably 2.00 to 10.00, and more preferably 3.00 to 8.00. The compound having the formula (5) may be used singly or in combination of two or more thereof. In addition, either acrylic acid or methacrylic acid may be used alone, or both may be used in combination.

In the reaction, a catalyst may be optionally used. Examples of the catalyst include amines such as triethylamine, triethylenediamine, bis-(2-dimethylaminoethyl) ether, and N-methylmorpholine; phosphines such as triphenylphosphine and tri(o-tolyl)phosphine; quaternary ammonium salts such as tetrabutylammonium chloride, benzyltriethylammonium chloride, and tetraethylhydroxylammonium; and imidazoles such as imidazole and 2-ethyl-4-methylimidazole.

The catalyst is usually used in a catalytic amount, which is preferably 0.1 to 20 mol% with respect to the compound having the formula (5). The catalyst may be used singly or in combination with two or more thereof.

In the reaction, a polymerization inhibitor may be optionally used. Examples of the polymerization inhibitor that can be used include various phenols, hydroquinones, benzoquinones, catechols, hydroxylamines, nitroso compounds. The amount of the polymerization inhibitor used is not particularly limited, but is preferably 0.001 to 10 wt%, more preferably 0.01 to 5 wt% with respect to acrylic acid or methacrylic acid.

After completion of the reaction, a solvent is optionally added, and the organic layer is washed with water and then heated under reduced pressure to distill off the solvent, whereupon the compound having the formula (1) is obtained. In addition, at the time of water washing, an aqueous solution of a metal hydroxide such as sodium hydroxide or potassium hydroxide, a metal carbonate such as sodium carbonate, sodium hydrogen carbonate, or potassium carbonate, or a metal hydrogen carbonate may be optionally used.

### [Resin composition]

The resin composition of the present invention includes (A) the compound having the formula (1) described above and (B) a polymerization initiator.

### [(B) Polymerization initiator]

A known polymerization initiator can be used herein, and examples thereof include a thermal radical generator and a photoradical generator.

Examples of the thermal radical generator include organic peroxides such as 1,1-bis(t-butylperoxy)2-methylcyclohexane, 1,1-bis(t-hexylperoxy)-3,3,5-trimethylcyclohexane, 1,1-bis(t-hexylperoxy)cyclohexane, 1,1-bis(t-butylperoxy)-3,3,5-trimethylcyclohexane, 1,1-bis(t-butylperoxy)cyclohexane, 2,2-bis(4,4-di-butylperoxycyclohexyl)propane, 1,1-bis(t-butylperoxy)cyclododecane, t-hexylperoxyisopropyl monocarbonate, t-butylperoxymaleic acid, t-butylperoxy-3,5,5-trimethylhexanoate, t-butylperoxylaurate, 2,5-dimethyl-2,5-di(m-toluoylperoxy)hexane, t-butylperoxyisopropyl monocarbonate, t-butylperoxy 2-ethylhexyl monocarbonate, t-hexylperoxybenzoate, 2,5-dimethyl-2,5-di(benzoylperoxy)hexane, t-butylperoxyacetate, 2,2-bis(t-butylperoxy)butane, t-butylperoxybenzoate, n-butyl-4,4-bis(t-butylperoxy)valerate, di-t-butylperoxyisophthalate, dicumyl peroxide, 2,5-dimethyl-2,5-di(t-butylperoxy)hexane, t-butylcumyl peroxide, di-t-butyl peroxide, p-menthane hydroperoxide, 2,5-dimethyl-2,5-di(t-butylperoxy)hexyne-3, diisopropylbenzene hydroperoxide, t-butyltrimethylsilyl peroxide, 1,1,3,3-tetramethylbutyl hydroperoxide, t-hexyl hydroperoxide, t-butyl hydroperoxide, and benzoyl peroxide; and azo compounds such as azobisisobutyronitrile, 1,1'-azobis(cyclohexane-1-carbonitrile), 2-(carbamoylazo)isobutyronitrile, 2-phenylazo-4-methoxy-2,4-dimethylvaleronitrile, azodi-t-octane, azodi-t-butane, and 2,2'-azobis[N-(2-propenyl)-2-methylpropionamide].

Examples of the photoradical generator include alkylphenone-type initiators such as 1-hydroxycyclohexyl phenyl ketone, 2,2-dimethoxy-1,2-diphenylethane-1-one, 2-methyl-1[4-(methylthio)phenyl]-2-morpholinopropan-1-one, 2-hydroxy-2-methyl-1-phenylpropan-1-one, and 1-[4-(2-hydroxyethoxy)-phenyl]-2-hydroxy-2-methyl-1-propan-1-one; acylphosphine oxide-type initiators such as bis(2,4,6-trimethylbenzoyl)-phenylphosphine oxide; and oxime ester-type initiators such as Irgacure Oxe01 (BASF Japan Ltd.), Irgacure Oxe02 (BASF Japan Ltd.), Irgacure Oxe03 (BASF Japan Ltd.), Irgacure Oxe04 (BASF Japan Ltd.), N-1919T (ADEKA Corporation), NCI-730 (ADEKA Corporation), NCI-831E (ADEKA Corporation), and NCI-930 (ADEKA Corporation).

In the resin composition of the present invention, the content of component (B) is preferably 0.05 to 20 parts by weight, and more preferably 0.1 to 5 parts by weight per 100 parts by weight of component (A). If the content of component (B) is within the above range, sufficient curability is easily obtained, and deterioration of the physical properties of the cured film due to the polymerization initiator can be prevented. Note that the polymerization initiator serving as component (B) may be used singly or in combination with two or more thereof.

### [(C) Radically polymerizable compound]

The resin composition of the present invention may further contain, as a component (C), a radically polymerizable compound different from the component (A). The radically polymerizable compound serving as component (C) may cause a radical polymerization reaction with the (meth)acryloyl group of component (A), thereby further increasing the strength of the cured resin film. Specific examples of the radically polymerizable compound include a compound having a polymerizable ethylenically unsaturated bond, preferably a (meth)acrylic acid ester compound.

The radically polymerizable compound serving as component (C) is preferably a polymerizable compound having three or more ethylenically unsaturated bonds. Examples of such a polymerizable compound include trimethylolpropane tri(meth)acrylate, pentaerythritol tri(meth)acrylate, pentaerythritol tetra(meth)acrylate, dipentaerythritol penta(meth)acrylate, dipentaerythritol hexa(meth)acrylate, tripentaerythritol octa(meth)acrylate, tripentaerythritol hepta(meth)acrylate, tetrapentaerythritol deca(meth)acrylate, tetrapentaerythritol nona(meth)acrylate, tris(2-(meth)acryloyloxyethyl)isocyanurate, ethylene glycol-modified pentaerythritol tetra(meth)acrylate, ethylene glycol-modified dipentaerythritol hexa(meth)acrylate, propylene glycol-modified pentaerythritol tetra(meth)acrylate, propylene glycol-modified dipentaerythritol hexa(meth)acrylate, caprolactone-modified pentaerythritol tetra(meth)acrylate, and caprolactone-modified dipentaerythritol hexa(meth)acrylate.

In the resin composition of the present invention, the content of component (C) is 0 to 100 parts by weight, preferably 1.0 to 80 parts by weight, and more preferably 1.0 to 50 parts by weight per 100 parts by weight of component (A). If the content of component (C) is 0.5 parts by weight or more, sufficient curability is obtained, and if the content is 100 parts by weight or less, the proportion of component (A) in the composition does not decrease, allowing the cured product to sufficiently exert the effect of the present invention. The radically polymerizable compound serving as component (C) may be used singly or in combination with two or more thereof.

### [(D) Solvent]

The resin composition of the present invention may contain a solvent as the component (D) in order to improve its coating properties. The solvent serving as the component (D) is not particularly limited as long as the components (A) to (C) described above, the component (E) described later, and other additives are soluble therein.

The solvent is preferably an organic solvent, and specific examples thereof include ketones such as cyclohexanone, cyclopentanone, and methyl-2-n-pentylketone; alcohols such as 3-methoxybutanol, 3-methyl-3-methoxybutanol, 1-methoxy-2-propanol, and 1-ethoxy-2-propanol; ethers such as propylene glycol monomethyl ether, ethylene glycol monomethyl ether, propylene glycol monoethyl ether, ethylene glycol monoethyl ether, propylene glycol dimethyl ether, and diethylene glycol dimethyl ether; and esters such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, propylene glycol mono-tert-butyl ether acetate, and γ-butyrolactone.

In particular, ethyl lactate, cyclohexanone, cyclopentanone, propylene glycol monomethyl ether acetate, γ-butyrolactone, and mixed solvents thereof, all of which have an excellent ability to dissolve the polymerization initiator, are preferred as the solvent (D).

In the resin composition of the present invention, the content of component (D) is preferably 50 to 2000 parts by weight, more preferably 50 to 1000 parts by weight, and still more preferably 50 to 100 parts by weight per 100 parts by weight of the total of components (A) and (C), from the viewpoint of compatibility and viscosity of the resin composition. The solvent serving as component (D) may be used singly or in combination with two or more thereof.

### [(E) Antioxidant]

The resin composition of the present invention may contain an antioxidant as an additive. Inclusion of an antioxidant is effective for enhancing heat resistance. Examples of the antioxidant include hindered phenol compounds and hindered amine compounds.

Although the hindered phenol compounds are not particularly limited, preferred examples include the following:
1,3,5-trimethyl-2,4,6-tris(3,5-di-t-butyl-4-hydroxybenzyl)benzene (trade name: IRGANOX 1330), 2,6-di-t-butyl-4-methylphenol (trade name: Sumilizer BHT), 2,5-di-t-butyl-hydroquinone (trade name: Nocrac NS-7), 2,6-di-t-butyl-4-ethylphenol (trade name: Nocrac M-17), 2,5-di-t-pentylhydroquinone (trade name: Nocrac DAH), 2,2'-methylenebis(4-methyl-6-t-butylphenol) (trade name: Nocrac NS-6), 3,5-di-t-butyl-4-hydroxy-benzyl phosphonate-diethyl ester (trade name: IRGANOX 1222), 4,4'-thiobis(3-methyl-6-t-butylphenol) (trade name: Nocrac 300), 2,2'-methylenebis(4-ethyl-6-t-butylphenol) (trade name: Nocrac NS-5), 4,4'-butylidenebis(3-methyl-6-t-butylphenol) (trade name: Adeka Stab AO-40), 2-t-butyl-6-(3-t-butyl-2-hydroxy-5-methylbenzyl)-4-methylphenyl acrylate (trade name: Sumilizer GM), 2-[1-(2-hydroxy-3,5-di-t-pentylphenyl)ethyl]-4,6-di-t-pentylphenyl acrylate (trade name: Sumilizer GS), 2,2'-methylenebis[4-methyl-6-(α-methyl-cyclohexyl)phenol], 4,4'-methylenebis(2,6-di-t-butylphenol) (trade name: Seenox 226M), 4,6-bis(octylthiomethyl)-o-cresol (trade name: IRGANOX 1520L), 2,2'-ethylenebis(4,6-di-t-butylphenol), octadecyl-3-(3,5-di-t-butyl-4-hydroxyphenyl)propionate (trade name: IRGANOX 1076), 1,1,3-tris(2-methyl-4-hydroxy-5-t-butylphenyl)butane (trade name: Adeka Stab AO-30), tetrakis[methylene-(3,5-di-t-butyl-4-hydroxyhydrocinnamate)]methane (trade name: Adeka Stab AO-60), triethylene glycol bis[3-(3-t-butyl-5-methyl-4-hydroxyphenyl)propionate] (trade name: IRGANOX 245), 2,4-bis(n-octylthio)-6-(4-hydroxy-3,5-di-t-butylanilino)-1,3,5-triazine (trade name: IRGANOX 565), N,N'-hexamethylenebis(3,5-di-t-butyl-4-hydroxy-hydrocinnamide) (trade name: IRGANOX 1098), 1,6-hexanediol-bis[3-(3,5-di-t-butyl-4-hydroxyphenyl)propionate] (trade name: IRGANOX 259), 2,2-thio-diethylenebis[3-(3,5-di-t-butyl-4-hydroxyphenyl)propionate] (trade name: IRGANOX 1035), 3,9-bis[2-[3-(3-t-butyl-4-hydroxy-5-methylphenyl)propionyloxy]1,1-dimethylethyl]2,4,8,10-tetraoxaspiro[5.5]undecane (trade name: Sumilizer GA-80), tris(3,5-di-t-butyl-4-hydroxybenzyl) isocyanurate (trade name: IRGANOX 3114), bis(ethyl 3,5-di-t-butyl-4-hydroxybenzylphosphonate) calcium/polyethylene wax 50/50 mixture (trade name: IRGANOX 1425WL), isooctyl-3-(3,5-di-t-butyl-4-hydroxyphenyl)propionate (trade name: IRGANOX 1135), and 4,4'-thiobis(6-t-butyl-3-methylphenol) (trade name: Sumilizer WX-R), 6-[3-(3-t-butyl-4-hydroxy-5-methylphenyl)propoxy]-2,4,8,10-tetra-t-butyldibenzo[d,f][1,3,2]dioxaphosphepin (trade name: Sumilizer GP).

Although the hindered amine compounds are not particularly limited, preferred examples include the following: p,p'-dioctyldiphenylamine (trade name: IRGANOX 5057), phenyl-α-naphthylamine (trade name: Nocrac PA), poly(2,2,4-trimethyl-1,2-dihydroquinoline) (trade name: Nocrac 224, 224-S), 6-ethoxy-2,2,4-trimethyl-1,2-dihydroquinoline (trade name: Nocrac AW), N,N'-diphenyl-p-phenylenediamine (trade name: Nocrac DP), N,N'-di-β-naphthyl-p-phenylenediamine (trade name: Nocrac White), N-phenyl-N'-isopropyl-p-phenylenediamine (trade name: Nocrac 810NA), N,N'-diallyl-p-phenylenediamine (trade name: Nonflex TP), 4,4'-(α,α-dimethylbenzyl)diphenylamine (trade name: Nocrac CD), p,p-toluenesulfonylaminodiphenylamine (trade name: Nocrac TD), N-phenyl-N'-(3-methacryloxy-2-hydroxypropyl)-p-phenylenediamine (trade name: Nocrac G1), N-(1-methylheptyl)-N'-phenyl-p-phenylenediamine (trade name: Ozonon 35), N,N'-di-sec-butyl-p-phenylenediamine (trade name: Sumilizer BPA), N-phenyl-N'-1,3-dimethylbutyl-p-phenylenediamine (trade name: Antigene 6C), alkylated diphenylamine (trade name: Sumilizer 9A), dimethyl-1-(2-hydroxyethyl)-4-hydroxy-2,2,6,6-tetramethylpiperidine succinate polycondensate (trade name: Tinuvin 622LD), poly[[6-(1,1,3,3-tetramethylbutyl)amino]-1,3,5-triazine-2,4-diyl][(2,2,6,6-tetramethyl-4-piperidyl)imino]hexamethylene[(2,2,6,6-tetramethyl-4-piperidyl)imino]] (trade name: CHIMASSORB 944), N,N'-bis(3-aminopropyl)ethylenediamine-2,4-bis[N-butyl-N-(1,2,2,6,6-pentamethyl-4-piperidyl)amino]-6-chloro-1,3,5-triazine condensate (trade name: CHIMASSORB 119FL), bis(1-octyloxy-2,2,6,6-tetramethyl-4-piperidyl)sebacate (trade name: Tinuvin 123), bis(2,2,6,6-tetramethyl-4-piperidyl)sebacate (trade name: Tinuvin 770), bis(1,2,2,6,6-pentamethyl-4-piperidyl) 2-(3,5-di-t-butyl-4-hydroxybenzyl)-2-n-butylmalonate (trade name: Tinuvin 144), bis(1,2,2,6,6-pentamethyl-4-piperidyl)sebacate (trade name: Tinuvin 765), tetrakis(1,2,2,6,6-pentamethyl-4-piperidyl) 1,2,3,4-butanetetracarboxylate (trade name: LA-57), tetrakis(2,2,6,6-tetramethyl-4-piperidyl) 1,2,3,4-butanetetracarboxylate (trade name: LA-52), an esterified mixture of 1,2,3,4-butanetetracarboxylic acid with 1,2,2,6,6-pentamethyl-4-piperidinol and 1-tridecanol (trade name: LA-62), an esterified mixture of 1,2,3,4-butanetetracarboxylic acid with 2,2,6,6-tetramethyl-4-piperidinol and 1-tridecanol (trade name: LA-67), an esterified mixture of 1,2,3,4-butanetetracarboxylic acid with 1,2,2,6,6-pentamethyl-4-piperidinol and 3,9-bis(2-hydroxy-1,1-dimethylethyl)-2,4,8,10-tetraoxaspiro[5.5]undecane (trade name: LA-63P), an esterified mixture of 1,2,3,4-butanetetracarboxylic acid with 2,2,6,6-tetramethyl-4-piperidinol and 3,9-bis(2-hydroxy-1,1-dimethylethyl)-2,4,8,10-tetraoxaspiro[5.5]undecane (trade name: LA-68LD), (2,2,6,6-tetramethylene-4-piperidyl)-2-propylene carboxylate (trade name: Adeka Stab LA-82), and (1,2,2,6,6-pentamethyl-4-piperidyl)-2-propylene carboxylate (trade name: Adeka Stab LA-87).

In the resin composition of the present invention, the content of component (E) is not particularly limited as long as the effect of the present invention is not impaired, but when component (E) is contained, the content thereof is preferably 0.01 to 1 wt% in the resin composition of the present invention. The antioxidant serving as component (E) may be used singly or in combination with two or more thereof.

### [Other additives]

The resin composition of the present invention may contain other additives in addition to the above-described components. Examples of the additives include surfactants commonly used for improving coating properties.

The surfactant is preferably a nonionic surfactant, and examples thereof include fluorochemical surfactants, specifically perfluoroalkyl polyoxyethylene ethanols, fluorinated alkyl esters, perfluoroalkylamine oxides, and fluorine-containing organosiloxane-based compounds. These surfactants are commercially available, and examples thereof include Fluorad (registered trademark) FC-430 (manufactured by 3M), Surflon (registered trademark) S-141 and S-145 (manufactured by AGC Seimi Chemical Co., Ltd.), Unidyne (registered trademark) DS-401, DS-4031, and DS-451 (manufactured by Daikin Industries Ltd.), Megaface (registered trademark) F-8151 (manufactured by DIC Corp.), and X-70-093 (manufactured by Shin-Etsu Chemical Co., Ltd). Among them, Fluorad FC-430 and X-70-093 are preferred. In the resin composition of the present invention, the content of the surfactant is not particularly limited as long as the effect of the present invention is not impaired, but when the surfactant is contained, the content thereof is preferably 0.01 to 1 wt% in the resin composition of the present invention.

As the additive, a silane coupling agent can also be used. Inclusion of a silane coupling agent is effective for enhancing the adhesion of the resin composition to an adherend. Examples of the silane coupling agent include epoxy silane coupling agents and aromatic-containing aminosilane coupling agents. The silane coupling agent can be used singly or in combination of two or more thereof. In the resin composition of the present invention, the content of silane coupling agent is not particularly limited as long as the effect of the present invention is not impaired, but when the silane coupling agent is contained, the content thereof is preferably 0.01 to 5 wt% in the resin composition of the present invention.

The method for preparing the resin composition of the present invention is not particularly limited, and examples thereof include a method of stirring and mixing the respective components, followed, if necessary, by filtration through a filter or the like to remove any solid content.

### [Cured product]

The cured product of the present invention is obtained from the resin composition. When the resin composition contains only a thermal radical generator as a polymerization initiator, a cured product can be obtained by a method including the steps of:
(i) forming a resin film on a substrate using the resin composition described above; and
(ii) heating the resin film.

Step (i) involves forming a resin film on a substrate using the resin composition. Examples of the substrate include a silicon wafer, a glass wafer, a quartz wafer, a plastic circuit board, and a ceramic circuit board.

The resin film can be formed by a known method. For example, the resin film can be formed by applying the resin composition onto a substrate by a coating technique such as dipping, spin coating or roll coating. The coating weight may be appropriately selected according to the purpose, preferably an amount at which the film thickness is 0.1 to 100 µm.

At this point, in order to efficiently perform the next curing reaction, the solvent and the like may be evaporated in advance by prebaking, if necessary. The prebaking can be performed, for example, at 40 to 160°C for about 1 minute to 1 hour.

Step (ii) involves heating and curing the resin film. The heating conditions are appropriately selected according to the types of the fluorene skeleton-containing (meth)acrylate compound, thermal radical generator, and radically polymerizable compound to be used, but the heating is preferably performed at 50 to 250°C for about 10 minutes to 6 hours.

When a photoradical generator is contained as a polymerization initiator, the resin composition of the present invention has photosensitivity. At this time, a cured product having a fine pattern can be produced by the method including:
(i) a step of forming a resin film on a substrate using the resin composition described above;
(ii) a step of performing exposure using the resin film; and
(iii) developing the exposed resin film using a developer.

Step (i) is the same as described above.

Next, in step (ii), the resin film is exposed to radiation. At this time, the exposure radiation is preferably of wavelength from 240 to 500 nm. Examples of the radiation include radiation of various wavelengths from radiation-emitting units, specifically UV radiation such as g-line or i-line and deep UV (248 nm). The exposure dose is preferably 10 to 5000 mJ/cm².

The exposure may be performed through a photomask. The photomask may be, for example, perforated with a desired pattern. Although the material of the photomask is not particularly limited, a material capable of shielding radiation of wavelength from 240 to 500 nm is preferred, for example, chromium.

Step (iii) following the exposure is of developing the resin film in a developer. The developer is preferably an organic solvent-based developer to be used as a solvent, for example, isopropyl alcohol, propylene glycol monomethyl ether, and propylene glycol monomethyl ether acetate. Development using the organic solvent-based developer gives a negative pattern in which an unexposed region of the resin film has been dissolved and removed. Development may be performed in a conventional manner, for example, by immersing a substrate on which a pattern has been formed in the developer. Thereafter, washing, rinsing, drying, and the like are performed, if necessary, to give a film having a desired pattern.

Although the patterning process is as described above, when there is no need for patterning, such as in the case where it is simply desired to form a uniform film using a resin composition containing a photoradical generator, film formation may be performed by, in step (ii) of the patterning process, exposure to radiation of a suitable wavelength without an intervening photomask.

The cured film produced by the method gives a cured product excellent in heat resistance and refractive index. The elongation percentage of the cured product is preferably 70% or more, more preferably 80% or more, and still more preferably 90% or more.

### EXAMPLES

Hereinafter, the present invention is specifically described with reference to Examples and Comparative Examples, but the present invention is not limited to the following Examples.

### [1] Synthesis of fluorene skeleton-containing (meth)acrylate compound

Compounds used in the synthesis are shown below.

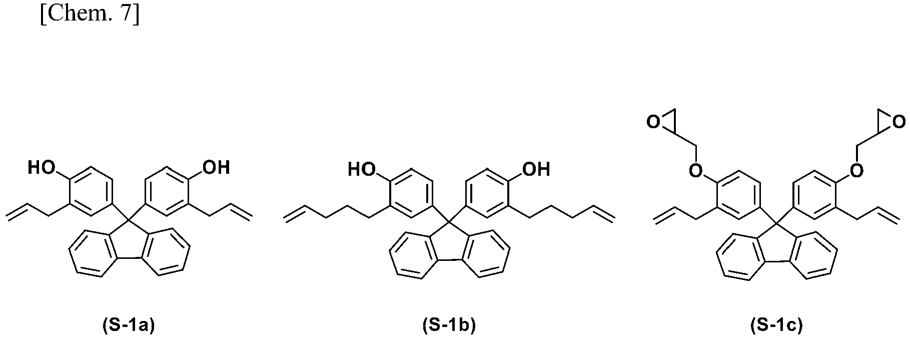

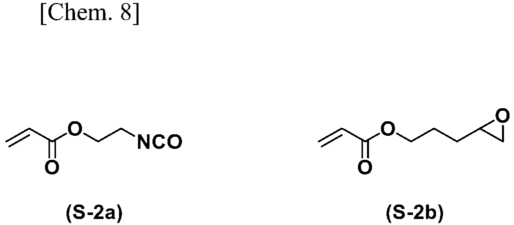

### [Example 1-1] Synthesis of Compound 1

In a 10-L flask equipped with a stirrer, a thermometer, a nitrogen purging system, and a reflux condenser, 430.6 g (1.00 mol) of the compound having the formula (S-la) was dissolved in 1800 g of propylene glycol monomethyl ether acetate. Furthermore, 282.2 g (2.00 mol) of the compound having the formula (S-2a) and 12.2 g (0.10 mol) of N,N-dimethyl-4-aminopyridine were added, then the mixture was heated to 60°C and stirred for 8 hours. After the reaction solution was allowed to cool to room temperature, followed by washing with pure water, the organic layer was heated and concentrated under reduced pressure to give Compound 1. Compound 1 was found to have the formula (A1) based on ultraviolet-visible absorption spectrum and ¹H-NMR (manufactured by Bruker Corp) analysis.

### ¹H-NMR (CDCl₃):

δ = 3.22 (4H, d, J=6.3 Hz), 3.51(4H, t, J=7.1Hz), 4.39 (4H, t, J=7.1Hz),
4.75-4.90 (4H, m), 5.76 (2H, ddt, J=17.1, 10.6, 6.3Hz), 6.02 (2H, dd, J=10.9, 1.5Hz),
6.33 (2H, dd, J=17.0, 1.5Hz), 6.51 (2H, dd, J=1.1, 0.5Hz), 6.65 (2H, br),
6.86 (2H, dd, J=17.0, 10.9Hz), 7.04 (2H, dd, J=8.7, 1.1Hz),
7.24 (2H, dd, J=8.7, 0.5Hz), 7.31-7.45 (4H, m), 7.64 (2H, dd, J=8.1, 1.4),
7.85 (2H, dd, J=8.5, 1.5Hz).

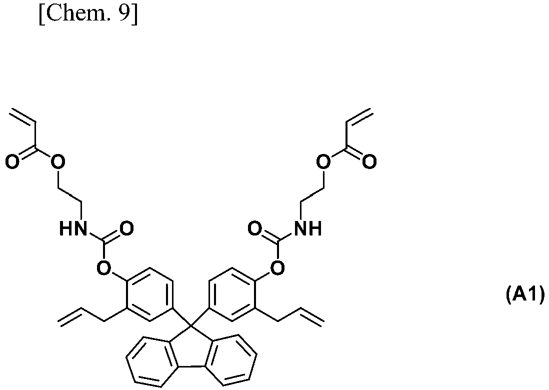

### [Example 1-2] Synthesis of Compound 2

In a 10-L flask equipped with a stirrer, a thermometer, a nitrogen purging system, and a reflux condenser, 486.7 g (1.00 mol) of the compound having the formula (S-1b) was dissolved in 1800 g of propylene glycol monomethyl ether acetate. Furthermore, 312.4 g (2.00 mol) of the compound having the formula (S-2b) and 20.2 g (0.20 mol) of triethylamine were added, then the mixture was heated to 80°C and stirred for 10 hours. The reaction solution was allowed to cool to room temperature, an aqueous solution of sodium hydrogen carbonate was added, the mixture was washed with pure water, and then the organic layer was heated and concentrated under reduced pressure to give Compound 2. Compound 2 was found to have the formula (A2) based on ultraviolet-visible absorption spectrum and ¹H-NMR (manufactured by Bruker Corp) analysis.

### ¹H-NMR (CDCl₃):

δ = 1.47-1.70 (8H, m), 1.85-2.04 (8H, m), 2.58-2.69 (4H, m), 2.97 (2H, br),
3.70-3.81 (2H, m), 3.96-4.08 (4H, m), 4.20-4.31 (4H, m), 4.73-4.88 (4H, m),
5.64 (2H, ddt, 1=16.9, 10.4, 7.4Hz), 6.02 (2H, dd, J=10.9, 1.5Hz),
6.37 (2H, dd, J=17.0, 1.5Hz), 6.75-6.95 (6H, m), 7.06 (2H, dd, J=8.4, 1.3Hz),
7.31-7.45 (4H, m), 7.64 (2H, dd, J=8.1, 1.4Hz), 7.85 (2H, dd, J=8.5, 1.5Hz).

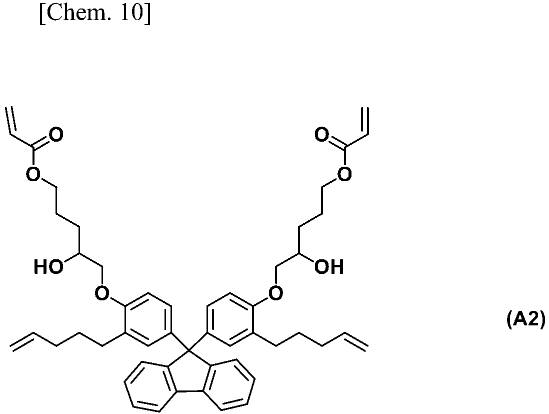

### [Example 1-3] Synthesis of Compound 3

In a 10-L flask equipped with a stirrer, a thermometer, a nitrogen purging system, and a reflux condenser, 542.7 g (1.00 mol) of the compound having the formula (S-1c) was dissolved in 1800 g of propylene glycol monomethyl ether. Furthermore, 516.4 g (6.00 mol) of methacrylic acid and 22.8 g (0.10 mol) of benzyltriethylammonium chloride were added, then the mixture was heated to 100°C and stirred for 12 hours. The reaction solution was allowed to cool to room temperature, 1000 g of methyl isobutyl ketone was added, followed by addition of an aqueous solution of sodium hydrogen carbonate, the mixture was allowed to cool and washed with pure water, and then the organic layer was heated and concentrated under reduced pressure to give Compound 3. Compound 3 was found to have the formula (A3) based on ultraviolet-visible absorption spectrum and ¹H-NMR (manufactured by Bruker Corp) analysis.

### 1H-NMR (CDCl₃):

δ = 1.86 (6H, s), 2.78 (2H, br), 3.15-3.26 (4H, m), 3.93-4.04 (4H, m),
4.10-4.12 (2H, m), 4.47-4.59 (4H, m), 4.76-4.91 (4H, m), 5.56 (2H, d, J=3.9Hz),
5.75 (2H, ddt, J=17.1, 10.6, 6.3Hz), 6.05 (2H, d, J=3.9Hz), 6.76-6.88 (4H, m),
7.06 (2H, dd, J=8.4, 1.3Hz), 7.31-7.45 (4H, m), 7.64 (2H, dd, J=8.1, 1.4Hz),
7.85 (2H, dd, J=8.5, 1.5Hz).

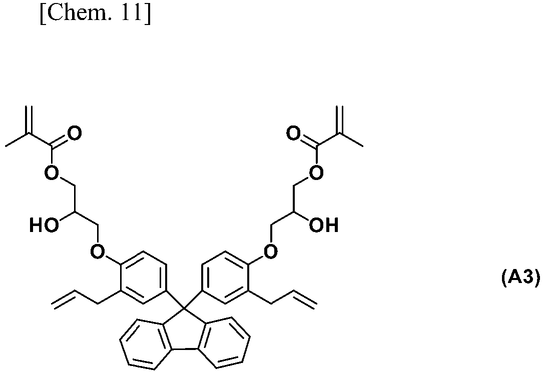

### [Comparative Example 1-1]

Comparative Compound 1 having the formula (B1),
9,9-bis(methacryloxyphenyl)-fluorene was synthesized according to the method described in Synthesis Example 1 of JP 3130555.

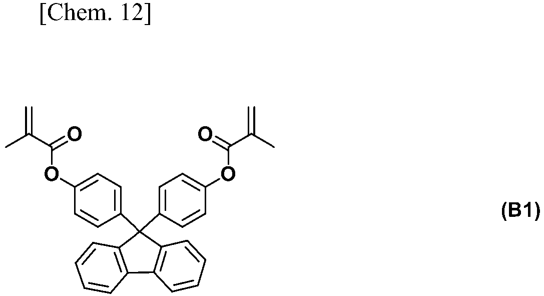

### [Comparative Example 1-2]

Comparative Compound 2 having the formula (B2),
9,9-bis(methacryloxydiethoxyphenyl)-fluorene was synthesized according to the method described in Synthesis Example 2 of JP 3130555.

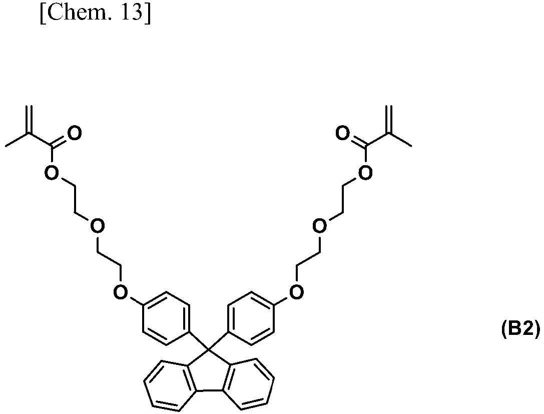

### [2] Preparation and evaluation of resin composition

### [Examples 2-1 to 2-6 and Comparative Examples 2-1 to 2-4]

The respective components were mixed and dissolved until uniform to achieve the composition shown in Table 1, and then microfiltration was performed with a 0.2 µm filter made of Teflon (registered trademark) to prepare a resin composition.

**[Table 1]**

| | | Example | | | | | | Comparative Example | | | |
|---|---|---|---|---|---|---|---|---|---|---|---|
| | | 2-1 | 2-2 | 2-3 | 2-4 | 2-5 | 2-6 | 2-1 | 2-2 | 2-3 | 2-4 |
| (A) | Compound 1 | 100 | 100 | - | - | - | - | - | - | - | - |
| | Compound 2 | - | - | 100 | 100 | - | - | - | - | - | - |
| | Compound 3 | - | - | - | - | 100 | 100 | - | - | - | - |
| | Comparative Compound 1 | - | - | - | - | - | - | 100 | 100 | - | - |
| | Comparative Compound 2 | - | - | - | - | - | - | - | - | 100 | 100 |
| (B) | Irgacure OXE01 | 3 | 3 | 3 | 3 | 3 | 3 | 3 | 3 | 3 | 3 |
| (C) | Dipentaerythritol hexaacrylate | - | 30 | - | 30 | - | 30 | - | 30 | - | 30 |
| (D) | Cyclopentanone | 55 | 55 | 55 | 55 | 55 | 55 | 55 | 55 | 55 | 55 |

### [Measurement of elongation percentage]

Each resin composition was applied to a polyethylene terephthalate film (38 µm) as a supporting substrate using a Baker type applicator SA-201 manufactured by Tester Sangyo Co., Ltd., and heated at 120°C for 3 minutes to remove the solvent, thereby preparing a film (thickness: 20 µm). Each of the obtained films was irradiated with radiation having a wavelength of 365 nm at 2000 mJ to prepare a cured product. A JIS No. 5 dumbbell-shaped test piece was punched out from the obtained cured product, the film of the supporting substrate was peeled off, and then a tensile test was performed using Strograph V10-D manufactured by Toyo Seiki Seisaku-sho, Ltd. (stretching speed: 50 mm/min). For each composition, five test pieces were measured, the elongation percentage was calculated from the broken distance and the initial distance to obtain the average value thereof.

**[Table 2]**

| | Example | | | | | | Comparative Example | | | |
|---|---|---|---|---|---|---|---|---|---|---|
| | 2-1 | 2-2 | 2-3 | 2-4 | 2-5 | 2-6 | 2-1 | 2-2 | 2-3 | 2-4 |
| Elongation percentage (%) | 91 | 89 | 96 | 95 | 95 | 93 | 56 | 52 | 61 | 61 |

From the above results, it was shown that the fluorene skeleton-containing (meth)acrylate compound of the present invention can improve the elongation of a cured product as compared with a fluorene skeleton-containing (meth)acrylate compound that has been conventionally used.

## Claims

1. A fluorene skeleton-containing (meth)acrylate compound comprising an alkenyl group having a double bond at a terminal thereof.

2. The fluorene skeleton-containing (meth)acrylate compound according to claim 1, which has the following formula (1): 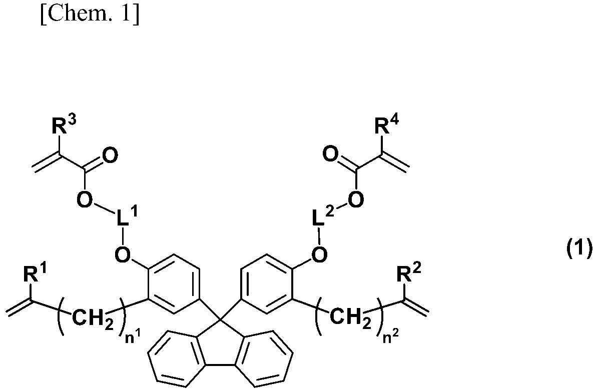 wherein n¹ and n² are each independently an integer of 1 to 7;
R¹ to R⁴ are each independently a hydrogen atom or a methyl group; and
L¹ and L² are each independently a saturated hydrocarbylene group having 1 to 15 carbon atoms, where a part of -CH₂- of the saturated hydrocarbylene group may be substituted with -O-, -S-, -SO₂-, -CO-, or -CONH-, and a part or all of hydrogen atoms of the saturated hydrocarbylene group may be substituted with a hydroxy group.

3. The fluorene skeleton-containing (meth)acrylate compound according to claim 2, wherein L¹ and L² are both saturated hydrocarbylene groups in which one or more hydrogen atoms are substituted with a hydroxy group or saturated hydrocarbylene groups in which one or more -CH₂- are substituted with -CONH-.

4. The fluorene skeleton-containing (meth)acrylate compound according to claim 2, wherein n¹ and n² are both 1.

5. The fluorene skeleton-containing (meth)acrylate compound according to claim 2, wherein both L¹ and L² have 1 to 8 carbon atoms.

6. A resin composition comprising the fluorene skeleton-containing (meth)acrylate compound according to any one of claims 1 to 5 and a polymerization initiator.

7. A cured product obtained from the resin composition according to claim 6.
